# EUROPÄISCHE PATENTSCHRIFT

(11) **EP 0 774 469 B1**
(45) Veröffentlichungstag und Bekanntmachung des Hinweises auf die Patenterteilung: **31.07.2002**
(21) Anmeldenummer: 96117311.9
(22) Anmeldetag: 29.10.1996
(51) Int. Cl.: C07H 13/06, C11D 3/22

(54) **Acylierte Saccharosemonocarbonsäuren**
Acylated saccharose monocarboxylic acids
Acides monocarboxyliques de saccharose

(30) Priorität: 14.11.1995 DE 19542303
(43) Veröffentlichungstag der Anmeldung: 21.05.1997
(73) Patentinhaber: SÜDZUCKER AKTIENGESELLSCHAFT MANNHEIM/OCHSENFURT, 68165 Mannheim (DE)
(72) Erfinder: Ehrhardt, Sonja, Dr., 64521 Gross-Gerau (DE); Kunz, Markwart, Dr., 67550 Worms (DE); Munir, Mohammed, Dr., 67271 Kindenheim (DE)
(74) Vertreter: Schrell, Andreas, Dr.

(56) Entgegenhaltungen:
- WO-A-80/00452
- DE-A- 1 467 705
- DE-A- 2 022 880
- DE-A- 4 307 388
- US-A- 3 872 020

## Beschreibung

Die Erfindung betrifft Derivate von Saccharosemonocarbonsäuren, deren Herstellung und Verwendung als grenzflächenaktive Substanzen.

Saccharoseester finden aufgrund ihres amphiphilen Charakters vielfach Verwendung als industrielle Hilfs- und Zusatzstoffe. So werden Saccharoseaacetate im Waschmittelbereich als Bleichmittelaktivatoren und Saccharose-Fettsäureester als grenzflächenaktive Verbindungen eingesetzt. Diese Ester werden im technischen Maßstab hauptsächlich mittels einer Reihe unterschiedlicher Umesterungsverfahren synthetisiert. Im Lösungsmittelverfahren wird Saccharose in Gegenwart eines basischen Katalysators in einem Lösungsmittel wie Dimethylformamid oder Dimethylsulfoxid mit Fettsäuremethylestern umgesetzt (JP 04,247,095). Im Mikroemulsionsverfahren wird der Fettsäureester in einer Lösung des Kohlenhydrats mit Hilfe eines Emulgators dispergiert. Das Lösungsmittel wird entfernt bevor die Reaktion stattfindet (EP 0 254 376). Im lösungsmittelfreien Verfahren, dem sogenannten direkten Verfahren, wird der Fettsäuremethylester in der Schmelze mit Saccharose und einem basischen Katalysator umgesetzt (GB 1 399 053). In anderen Verfahren finden Lipasen (DE 34 30 944) Verwendung. Schließlich ist es bekannt, die Veresterung von Saccharose mit Carbonsäurechloriden beziehungsweise -anhydriden durchzuführen.

Die bekannten Umesterungsverfahren sind sowohl hinsichtlich ihrer Prozessführung als auch der Produktisolierung sehr aufwendig. Die Reaktionsparameter Druck und Temperatur müssen häufig variiert werden und zur Gewinnung der Saccharoseester muß mehrmals extrahiert und destilliert werden. Zudem können die hohen Reaktionstemperaturen in Kombination mit den langen Reaktionszeiten zu unerwünschter Verfärbung der Produkte führen. Die auf diese aufwendige Art erhaltenen Saccharose-Carbonsäureester zeigen zwar als Mono- beziehungsweise Diester grenzflächenaktive Eigenschaften, sind jedoch aufgrund ihrer begrenzten Löslichkeit in Wasser nur eingeschränkt als Tenside verwendbar. Die Monoester lösen sich schlecht in kaltem Wasser, während die Diester in Wasser nur emulgierbar sind.

Die gezielte Herstellung der drei möglichen Saccharosemonocarbonsäuren ist erst seit kurzem möglich (DE 43 07 388). Derivate der Saccharosemonocarbonsäuren sind mit Ausnahme der Acetate bisher noch nicht beschrieben worden. Auch bei den beschriebenen Derivaten anderer Kohlenhydratsäuren handelt es sich überwiegend um Acetate, die häufig als Synthesezwischenprodukte benötigt werden. Den meisten dieser acylierten Kohlenhydratsäuren ist gemeinsam, daß sie nur sehr geringe grenzflächenaktiven Eigenschaften aufweisen. Bei anderen ist darüber nichts bekannt.

Das der Erfindung zugrundeliegende technische Problem besteht somit in der Bereitstellung von Saccharosederivaten, die die vorgenannten Nachteile überwinden, insbesondere also als Tenside und Emulgatoren geeignet und einfach herzustellen sind.

Die Lösung dieses technischen Problems liegt in der Bereitstellung acylierter Saccharosemonocarbonsäuren gemäß Hauptanspruch, Verfahren zu deren Herstellung und deren Verwendung als grenzflächenaktive Substanzen. Die vorliegende Erfindung stellt demgemäß acylierte Saccharosemonocarbonsäure der Formel I 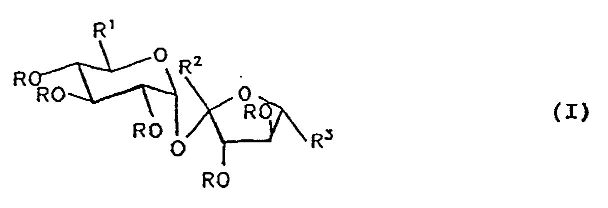 bereit, in welcher R¹, R² oder R³ = CH₂OH oder COOH, und R = H oder CO - R' bedeuten, wobei R' = (CH₂)ₙ - CH₃ mit n = 4 - 16 ist. Die Saccharosederivate der vorliegenden Erfindung zeichnen sich also dadurch aus, daß ein Rest aus R¹, R² oder R³ = COOH ist, während die jeweiligen beiden anderen Reste aus R¹, R² oder R³ = CH₂OH sind und zumindest ein Rest R = CO-R' mit R' = (CH₂)ₙ - CH₃ mit n = 4 - 16 ist. Die vorliegende Erfindung stellt insbesondere mit längerkettigen aliphatischen Carbonsäuren C₆ bis C₁₈ acylierte 1-O-(β-D-Fructofuranuronyl)-α-D-glucopyranoside (C₆-Saccharosemonocarbonsäure), 2-Keto-6-O-(a-D-glucopyranosyl)-β-glucofuranonsäuren (C₁-Saccharosemonocarbonsäure), 1-O-(β-D-Fructofuranosyl)-α-D-glucopyranuronide (C_{6'}-Saccharosemonocarbonsäure) oder deren Gemische bereit.

Diese Verbindungen weisen ausgezeichnete grenzflächenaktive Eigenschaften auf und sind daher vielfältig als industrielle Hilfs- und Zusatzstoffe, beispielweise als Tenside, Emulgatoren, Stabilisatoren, Weichmacher oder Solubilisatoren verwendbar.

Zur Veresterung der Saccharosemonocarbonsäuren oder deren Gemisch kann sowohl eine bestimmte Fettsäure als auch ein Gemisch aus mehreren unterschiedlichen langkettigen aliphatischen Fettsäuren C₆ bis C₁₈ verwendet werden. Demgemäß kann die erfindungsgemäß acylierte Saccharosemonocarbonsäure unterschiedliche Reste R' tragen. In einer bevorzugten Ausführungsform betrifft die Erfindung insbesondere eine acylierte Saccharosemonocarbonsäure, in der -CO- R' ein Capronsäure-, Caprylsäure-, Caprinsäure-, Laurinsäure-, Myristinsäure-, Palmitinsäure- oder Stearinsäurerest ist.

Die Erfindung betrifft ferner wässrige Lösungen, Seifen, Reinigungsmittel wie Scheuermittel oder Waschmittel (auch Haarwaschmittel, Vollwaschmittel, Badezusatzmittel), Lebensmittel, Kosmetika, Emulsionen, Suspensionen, Gelees, Cremes, Pasten und Pulver, die mindestens eine der acylierten Saccharosemonocarbonsäuren der Erfindung enthalten.

Insbesondere betrifft die Erfindung ein Scheuermittel, das 85 Gew.-% bis 95 Gew.-% Quarzmehl, 1 Gew.-% bis 5 Gew.-% Alkalien (z.B. Soda, Borax), 1 Gew.-% bis 5 Gew.-% Polyphosphate, 1 Gew.-% bis 5 Gew.-% der erfindungsgemäßen Verbindungen oder deren Gemische und 0 Gew.-% bis 1 Gew.-% Aktiv-Chlor-Verbindungen enthalten. Die Erfindung betrifft auch ein Waschmittel, insbesondere ein Vollwaschmittel, das 10 Gew.-% bis 25 Gew.-% der erfindungsgemäßen Verbindungen oder deren Gemische, 20 Gew.-% bis 60 Gew.-% Komplexbildner, 0 Gew.-% bis 30 Gew.-% Bleichmittel, 0 Gew.-% bis 2 Gew.-% Bleichmittelaktivatoren, 0 Gew.-% bis 5 Gew.-% Korrosionsinhibitoren, 0 Gew.-% bis 0,3 Gew.-% optische Aufheller, 0 Gew.-% bis 2 Gew.-% Vergrauungsinhibitoren, 0 Gew.-% bis 1 Gew.-% Enzyme, 0 Gew.-% bis 0,2 Gew.-% Parfüme und 0 Gew.-% bis 30 Gew.-% Füllstoffe enthält. Die Erfindung betrifft auch Kosmetika wie zum Beispiel Deoroller, die 30 Gew.-% bis 60 Gew.-% Alkohol, 0 Gew.-% bis 1 Gew.-% Duftstoffe, 0,1 Gew.-% bis 1 Gew.-% Deowirkstoffe, 0,5 Gew.-% bis 5 Gew.-% der erfindungsgemäßen Verbindungen oder deren Gemische und Wasser enthalten.

Die erfindungsgemäßen Verbindungen können in verschiedenen Applikationsformen beispielsweise im Lebensmittel-, Arzneimittel- oder Hygienebereich eingesetzt werden. Die Applikationsform ist dabei dem jeweiligen Anwendungsbereich anzupassen.

Die Erfindung betrifft außerdem ein Verfahren zur Herstellung der acylierten Saccharosemonocarbonsäuren der Formel I, wobei Saccharosemonocarbonsäuren der Formel II 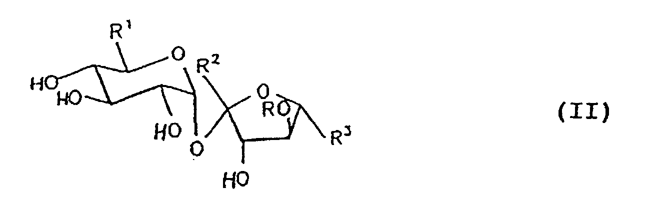 oder eines Gemisches davon in Pyridin mit Carbonsäureanhydrid oder -chlorid umgesetzt werden, dessen Carbonsäurerest = -CO-R' ist, wobei R', R¹, R², R³ und n die vorstehend genannte Bedeutung haben. In einer bevorzugten Ausführungsform betrifft die Erfindung ein Verfahren zur Herstellung der acylierten Saccharosemonocarbonsäuren, in dem 0,003 bis 0,03 Mol Saccharosemonocarbonsäuren entweder als Einzelkomponente oder als deren Gemisch in Pyridin mit 0,003 bis 0,5 Mol Carbonsäureanhydriden beziehungsweise -chloriden, die rein oder gelöst in Chloroform eingesetzt werden können, mit beziehungsweise ohne Katalysator umgesetzt werden.

Die Erfindung betrifft in einer weiteren Ausgestaltung auch ein Verfahren zur Herstellung der erfindungsgemäßen Verbindungen, in dem die Umsetzung der Saccharosemonocarbonsäuren der Formel II oder deren Gemische lösungsmittelfrei, insbesondere in Gegenwart von Kaliumcarbonat und Kaliumstearat, mit Carbonsäureestern, vorzugsweise Methyl- oder Ethylestern, vorzugsweise deren Fettsäureestern, erfolgt. Besonders bevorzugt werden in dieser Ausgestaltung der Erfindung 0,003 Mol bis 0,02 Mol Fettsäureester und 0,003 Mol bis 0,03 Mol der Saccharosemonocarbonsäure oder deren Gemisch eingesetzt.

Das Verhältnis Hydrophilie zu Hydrophobie, das für die Eignung der erfindungsgemäßen Verbindungen als grenzflächenaktive Substanzen besonders bedeutsam ist, wird über den Alkylsubstituenten und den Acylierungsgrad eingestellt. Der Acylierungs- oder Substitutionsgrad der erfindungsgemäßen Verbindung gibt an, wieviele der vorhandenen Hydroxygruppen acyliert sind. Der Acylierungsgrad kann über das Molverhältnis des aliphatischen Carbonsäurederivats zur Saccharosemonocarbonsäure eingestellt werden und ist als Mittelwert angegeben. Die erfindungsgemäß verwendeten Saccharosemonocarbonsäuren weisen sieben freie Hydroxygruppen auf, die achte Hydroxygruppe liegt als Carboxylat vor. Der Aylierungsgrad kann demnach zwischen 1 und 7 liegen. Erfindungsgemäß bevorzugt liegt der Acylierungsgrad bei ≤ 3, besonders bevorzugt bei ≤ 2.

In einer besonders bevorzugten Ausführungsform der Erfindung werden zur Herstellung der acylierten Saccharosemonocarbonsäuren als Anhydride beziehungsweise Säurechloride bevorzugt Verbindungen eingesetzt, deren Carbonsäurerest ein Capron-, Capryl-, Caprin-, Laurin-, Myristin-, Palmitin- oder Stearinsäurerest ist. Die Acylierung erfolgt bei der Verwendung von Anhydriden oder Chloriden insbesondere bei Temperaturen zwischen 0°C und 100°C, bevorzugt zwischen 20°C und 50°C.

In einer weiteren bevorzugten Ausführungsform der Erfindung werden zur lösungsmittelfreien Herstellung der acylierten Saccharosemonocarbonsäuren als Ethyl- oder Methylester insbesondere Ester von Fettsäuren, bevorzugt der Caprinsäure, Laurinsäure, Myristinsäure, Palmitinsäure und Stearinsäure eingesetzt. Die Acylierung erfolgt bei der lösungsmittelfreien Herstellung der erfindungsgemäßen Verbindung insbesondere zwischen 100°C und 200°C, bevorzugt zwischen 120°C und 180°C.

Die Erfindung betrifft auch die vorgenannten Verfahren, in denen zusätzlich ein Katalysator verwendet wird. Als Katalysator wird insbesondere 4-(Dimethylamino)-pyridin oder Kaliumcarbonat mit Kaliumstearat verwendet.

Die durch die erfindungsgemäßen Verfahren erhaltenen Verbindungen werden mittels spektroskopischer Verfahren wie IR, ¹H- und ¹³C-NMR analysiert. Die jeweiligen charakteristischen Daten lauten wie folgt:
IR-Spektrum: die Kohlenstoff-Wasserstoff-Valenzschwingung des Substituenten liegt bei 2.900 cm⁻¹, die Carbonylvalenzschwingung des Esters im Bereich 1.720 bis 1.750 cm⁻¹, die Carboxylat-Valenzschwingung im Bereich 1660 bis 1710 cm⁻¹, die Kohlenstoff-Sauerstoff-Valenzschwingung im Bereich 1.050 bis 1.250 cm⁻¹;
¹H-NMR-Spektrum: die Substituentensignale liegen bei 2,3 ppm und im Bereich 0,9 bis 1,6 ppm, die Kohlenhydratsignale in den Bereich 5,2 bis 5,7 ppm und im Bereich 3,4 bis 4,6 ppm;
¹³C-NMR: die Signale der Carbonylkohlenstoffatome liegen im Bereich 175 bis 180 ppm, die aliphatischen Substituentensignale im Bereich 14 bis 33 ppm, das Saccharosegerüst in den Bereichen 103 bis 105 ppm, 92 bis 94 ppm und 61 bis 83 ppm.

Der Acylierungsgrad der erfindungsgemäßen Verbindungen wird mittels des Protonenverhältnisses durch Integration des ¹H-NMR-Signals ermittelt.

Die folgenden Beispiele erläutern die Erfindung.

### Beispiel 1:

### Acylierung des 1-O-(β-D-Fructofuranuronyl)-α-D-glucopyranosids (C₆-Saccharosemonocarbonsäure) ohne Katalysator mit Capronsäurechlorid

In einem thermostatisierbaren Reaktor mit Rührwerk werden 10 g (0,03 Mol) C₆-Saccharosemonocarbonsäure in 500 mL Pyridin gelöst. Bei 0°C werden 8,2 mL (0,06 Mol) Capronsäurechlorid, gelöst in 8 mL Chloroform, zugetropft. Nach 24 h bei 25 °C wird bei 0°C die Reaktionsmischung mit 500 mL Wasser versetzt und unter Vakuum (15 hPa) eingeengt. Der Rückstand wird in 100 mL Wasser aufgenommen, die Lösung mit Essigsäureethylester (4 x 50 mL) extrahiert und lyophilisiert. Das Produkt wird als farbloser Feststoff in 60 Gew.-% Ausbeute erhalten (Acylierungsgrad 1). Die Oberfächenspannung einer 0.8 Gew.-%igen wäßrigen Lösung beträgt 54,3 mN/m.

### Beispiel 2:

### Acylierung der 2-Keto-6-O-(α-D-glucopyranosyl)-β-Dglucofuranonsäure (C₁-Saccharosemonocarbonsäure) ohne Katalysator mit Capronsäurechlorid.

In einem thermostatisierbaren Reaktor mit Rührwerk werden 5 g (0.014 Mol) C₁-Saccharosemonocarbonsäure in 250 mL Pyridin gelöst. Bei 0°C werden 4.1 mL (0.03 Mol) Capronsäurechlorid, gelöst in 4 mL Chloroform, zugetropft. Nach 24 h bei 25°C wird bei 0°C die Reaktionsmischung mit 250 mL Wasser versetzt und unter Vakuum (15 hPa) eingeengt. Der Rückstand wird in 75 mL Wasser aufgenommen, die Lösung mit Essigsäureethylester (4 x 30 mL) extrahiert und lyophilisiert. Das Produkt wird als farbloser Feststoff in 58 Gew.-% Ausbeute erhalten (Acylierungsgrad 1). Die Oberflächenspannung einer 0.8 Gew.-% wäßrigen Lösung beträgt 54.1 mN/m.

### Beispiel 3:

### Acylierung des 1-O-(β-D-Fructofuranosyl)-α-D-glucopyranuronids (C_{6'}-Saccharosemonocarbonsäure) ohne Katalysator mit Caprylsäurechlorid.

In einem thermostatisierbaren Reaktor mit Rührwerk werden 10 g (0.028 Mol) C_{6'}-Saccharosemonocarbonsäuren in 500 mL Pyridin gelöst. Bei 0°C werden 8.6 mL (0.06 Mol) Caprylsäurechlorid zugetropft. Nach 48 h bei 25°C wird bei 0°C die Reaktionsmischung mit 500 mL Wasser versetzt und unter Vakuum (15 hPa) eingeengt. Der Rückstand wird in 100 mL Wasser aufgenommen, die Lösung mit Essigsäureethylester (4 x 50 mL) extrahiert und lyophilisiert. Das Produkt wird als farbloser Feststoff in 55 Gew.-% Ausbeute erhalten (Acylierungsgrad 1). Die Oberflächenspannung einer 1.4 Gew.-% wäßrigen Lösung beträgt 29.5 mN/m.

### Beispiel 4:

### Acylierung der Saccharosemonocarbonsäuren ohne Katalysator mit Caprylsäurechlorid

In einem thermostatisierbaren Reaktor mit Rührwerk werden 10 g (0,03 Mol) Saccharosemonocarbonsäuren (C₆-, C₁- und C_{6'}-Säuren als Gemisch, Gewichtsverhältnis 45 : 10 : 45) in 500 mL Pyridin gelöst. Bei 0°C werden 8,6 mL (0,06 Mol) Caprylsäurechlorid zugetropft. Nach 48 h bei 25°C wird bei 0°C die Reaktionsmischung mit 500 mL Wasser versetzt und unter Vakuum (15 hPa) eingeengt. Der Rückstand wird in 100 mL Wasser aufgenommen, die Lösung mit Essigsäureethylester (4 x 50 mL) extrahiert und lyophilisiert. Das Produkt wird als farbloser Feststoff in 55 Gew.-% Ausbeute erhalten (Acylierungsgrad 1). Die Oberflächenspannung einer 1,6 Gew.-%igen wäßrigen Lösung beträgt 29,2 mN/m.

### Beispiel 5:

### Katalytische Acylierung der Saccharosemonocarbonsäuren mit Caprinsäureanhydrid

In einem thermostatisierbaren Reaktor mit Rührwerk werden 5 g (0,014 Mol) Saccharosemonocarbonsäuren (C₆-, C₁- und C_{6'}-Säuren als Gemisch, Gewichtsverhältnis 40 : 10 : 50) in 200 mL Pyridin gelöst und mit 0,9 g (0,007 Mol) 4-(Dimethylamino)-pryridin versetzt. Bei 0°C werden 18,4 mL (0,05 Mol) Caprinsäureanhydid zugetropft. Nach 48 h bei 40°C wird bei 0°C die Reaktionsmischung mit 200 mL Wasser versetzt und unter Vakuum (15 hPa) eingeengt. Der Rückstand wird in 100 mL Wasser aufgenommen, die Lösung mit Essigsäureethylester (5 x 50 mL) extrahiert und lyophilisiert. Das Produkt wird als leicht gefärbter Feststoff in 55 Gew.-% Ausbeute erhalten (Acylierungsgrad 1.5). Die Oberflächenspannung einer 0,7 Gew.-%igen wäßrigen Lösung beträgt 24,2 mN/m.

### Beispiel 6:

### Katalytische Acylierung der Saccharosemonocarbonsäuren mit Laurinsäurechlorid

In einem thermostatisierbaren Reaktor mit Rührwerk werden 5 g (0,014 Mol) Saccharosemonocarbonsäuren (C₆-, C₁- und C_{6'}-Säuren als Gemisch, Gewichtsverhältnis 50 : 5 : 45) in 200 mL Pyridin gelöst und mit 1,5 g (0,012 Mol) 4-(Dimethylamino)-pryridin versetzt. Bei 0°C werden 6,6 mL (0,03 Mol) Laurinsäurechlorid, gelöst in 7 mL Chloroform, zugetropft. Nach 48 h bei 25°C wird bei 0°C die Reaktionsmischung mit 200 mL Wasser versetzt und unter Vakuum (15 hPa) eingeengt. Der Rückstand wird in 100 mL Wasser aufgenommen, die Lösung mit Essigsäureethylester (5 x 50 mL) extrahiert und lyophilisiert. Das Produkt wird als leicht gefärbter Feststoff in 55 Gew.-% Ausbeute erhalten (Acylierungsgrad 1). Die Oberflächenspannung einer 1 Gew.-%igen wäßrigen Lösung beträgt 28,9 mN/m.

### Beispiel 7:

### Katalytische Acylierung der Saccharosemonocarbonsäuren mit Laurinsäurechlorid

In einem thermostatisierbaren Reaktor mit Rührwerk werden 5 g (0,014 Mol) Saccharosemonocarbonsäuren (C₆-, C₁- und C_{6'}-Säuren als Gemisch, Gewichtsverhältnis 50 : 5 : 45) in 200 mL Pyridin gelöst und mit 0,9 g (0,007 Mol) 4-(Dimethylamino)-pryridin versetzt. Bei 0°C werden 23,8 mL (0,1 Mol) Laurinsäurechlorid, gelöst in 25 mL Chloroform, zugetropft. Nach 48 h bei 25°C wird bei 0°C die Reaktionsmischung mit 200 mL Wasser versetzt und unter Vakuum (15 hPa) eingeengt. Der Rückstand wird in 100 mL Essigsäureethylester aufgenommen, die Lösung mit Wasser (4 x 25 mL) extrahiert, getrocknet und eingeengt. Das Produkt wird als leicht gefärbter Feststoff in 70 Gew.-% Ausbeute erhalten (Acylierungsgrad 5).

### Beispiel 8:

### Lösungsmittelfreie Acylierung der Saccharosemonocarbonsäuren mit Palmitinsäuremethylester

Über einen Doppelschneckenextruder wird eine Mischung aus 10 g (0.028 Mol) Saccharosemonocarbonsäuren (C₆-, C₁- und C_{6'}-Säuren als Gemisch, Gewichtsverhältnis 50 : 5 : 45), 5.4 g (0.022 Mol) Palmitinsäuremethylester, 0.81 g (0.002 Mol) Kaliumstearat und 0.15 g (0.001 Mol) Kaliumcarbonat in einen Reaktor extrudiert und 60 min lang bei 100 mBar auf 160°C erwärmt. Das erhaltene Rohprodukt wird in 100 mL Wasser suspendiert, filtriert, das Filtrat mit Diethylether (3 x 50 mL) extrahiert und anschließend gefriergetrocknet. In einer Soxhlet-Apparatur wird das Lyophilisat mit siedendem Butanol (300 mL) 8 h extrahiert. Aus der eingeengten Extraktphase läßt sich das Produkt als leicht gefärbter Feststoff in 45 Gew.-% Ausbeute erhalten (Acylierungsgrad 1). Die Oberflächenspannung einer 1 Gew.-%igen wäßrigen Lösung beträgt 35 mN/m.

### Beispiel 9:

### Bestimmung der Oberflächenspannung

Die erfindungsgemäßen Verbindungen, insbesondere die mit einem Acylierungsgrad ≤ 3, weisen grenzflächenaktive Eigenschaften auf. Tabelle 1 verdeutlicht, daß die erfindungsgemäßen Verbindungen die Oberflächenspannung von Wasser vermindern. Die Oberflächenspannung σ wäßriger Lösungen der erfindungsgemäßen acylierten Saccharosemonocarbonsäuren wurde bei 25°C nach der Wilhelmy-Methode gemessen. Die erfindungsgemäße Verbindung wird in Tabelle 1 in Kurzform als Salz der zur Acylierung verwendeten Fettsäure angegeben (DS bedeutet Substitutionsgrad, die Gew.-%-Angabe betrifft die Konzentration der Lösung).

**Tabelle 1:**

| Oberflächenspannung | |
|---|---|
| **Probe** | σ (mN/m) |
| Capronat (DS ≈1; 0.8 %) | 54.3 |
| Caprylat (DS≈ 1; 1.6 %) | 29.2 |
| Caprinat (DS 1.5; 0.7 %) | 24.2 |
| Laurat (DS ≈1; 1 %) | 28.9 |

Die durch die erfindungsgemäßen Verbindungen bewirkte Herabsetzung der Oberflächenspannung von Wasser macht diese Verbindungen besonders geeignet zum Einsatz in Wasch-, Spül- und Reinigungsmitteln sowie als Emulgatoren beispielsweise im Lebensmittel-, Süßmittel- und Arzneimittelbereich.

### Beispiel 10:

### Solubilisierung von Sudan Rot B in Wasser mit acylierten Saccharosemonocarbonsäuren

Lösungen acylierter Saccharosemonocarbonsäuren verschiedener Konzentrationen (0,06 g, 0,12 g und 0,24 g in 20 mL Wasser) wurden jeweils mit 0,01 g Sudan Rot B versetzt. Der Farbstoff wurde durch Ultraschall dispergiert. Die Suspension wurde anschließend 60 min bei 7.000 U/min zentrifugiert und membranfiltriert (regenerierte Zellulose, 0,2 µm). Die Extinktion der überstehenden klaren Lösung wurde photometrisch bei einer Wellenlänge von 516 nm (Küvettenlänge 1 cm) gemessen. Als Nullprobe diente eine Lösung von Sudan Rot B in Wasser (0,01 g in 20 mL), die wie die Esterlösung vorbehandelt wurde. In Tabelle II sind beispielhaft die Ergebnisse des Caprylats der Saccharosemonocarbonsäuren (Acylierungsgrad 1) angegeben.

**Tabelle 2:**

| Solubilisierung | |
|---|---|
| **Konzentration des Caprylats ox. Saccharose** (Gew.-%) Acylierungsgrad 1 | **Extinktion** |
| 0 | 0.0096 |
| 0.6 | 0.0654 |
| 1.2 | 0.1513 |

Die Tabelle zeigt, daß die erfindungsgemäßen Verbindungen ausgezeichnete Solubilisierungseigenschaften aufweisen. Die Verbindungen eignen sich dementsprechend beispielsweise für Wasch-, Spülund Reinigungsmittel.

### Beispiel 11 :

### Herstellung eines Haarwaschmittels

Zur Herstellung von 100 g eines Haarwaschmittels werden 10 g der erfindungsgemäßen Verbindungen (Laurat der C₆-, C₁- und C_{6'}-Saccharosemonocarbonsäuren), 15 g Tego-Betain, 2 g NaCl, 0,2 g Konservierungsmittel und 0,5 g Parfümöl gemischt und auf 100 g mit Wasser aufgefüllt. Man erhält ein haut- und haarverträgliches, hochwirksames Haarwaschmittel.

### Beispiel 12:

### Herstellung eines Scheuermittels:

Zur Herstellung von 100 g eines Scheuermittels werden 4 g der erfindungsgemäßen Verbindungen (Caprinat der C₆-, C₁- und C_{6'}-Saccharosemonocarbonsäuren) 2 g Octadecylpolyethylenglykolether, 5 g Pentanatriumtriphosphat, 1 g Duftstoff gemischt und mit Quarzmehl auf 100 g aufgefüllt.

### Beispiel 13:

### Herstellung eines Waschmittels:

Zur Herstellung von 100 g eines Waschmittels werden 20 g der erfindungsgemäßen Verbindungen (Laurat der C₆-, C₁- und C_{6'}-Saccharosemonocarbonsäuren), 20 g Komplexbildner (Zeolith), 0,5 g Proteasen, 4 g Natriumcitrat, 10 g Ethanol, gegebenenfalls Parfüm und Farbstoff, gemischt und mit Wasser auf 100 g aufgefüllt.

### Beispiel 14:

### Herstellung eines Badezusatzmittels:

Zur Herstellung von 100 g eines Badezusatzmittels werden 25 g der erfindungsgemäßen Verbindungen (Laurat der C₆-, C₁- und C_{6'}-Saccharosemonocarbonsäuren), 5 g Cocosfettsäureethanolamid, 6 g Mandelöl, 1 g Natriumchlorid, 0,4 g Konservierungsmittel, 1 g Hexadecanol, 2 g Parfüm gemischt und mit Wasser auf 100 g aufgefüllt.

## Patentansprüche

1. Acylierte Saccharosemonocarbonsäuren der Formel I 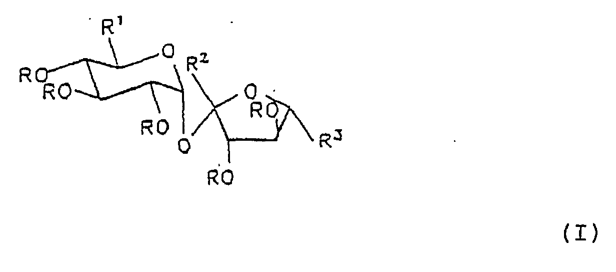 in welcher einer der Reste R¹, R² und R³ COOH darstellt und die beiden anderen Reste CH₂OH sind und R = H oder CO - R' bedeutet, wobei R' = (CH₂)ₙ - CH₃ mit n = 4 - 16 ist, und wobei zumindest ein Rest R = CO - R' ist.

2. Acylierte Saccharosemonocarbonsäuren nach Anspruch 1, **dadurch gekennzeichnet, daß** die acylierten Saccharosemonocarbonsäuren verschiedene Reste R' aufweisen.

3. Acylierte Saccharosemonocarbonsäuren nach Anspruch 1 oder 2, **dadurch gekennzeichnet, daß** -CO -R' ein Capronsäure-, Caprylsäure-, Caprinsäure-, Laurinsäure-, Myristin-, Palmitinsäure-, oder Stearinsäurerest ist.

4. Acylierte Saccharosemonocarbonsäuren nach einem der Ansprüche 1 bis 3, **dadurch gekennzeichnet, daß** diese einen Acylierungsgrad von ≤ 2 aufweisen.

5. Wässrige Lösung, enthaltend mindestens eine der acylierten Saccharosemonocarbonsäuren nach einem der Ansprüche 1 bis 4.

6. Pulver, enthaltend mindestens eine der acylierten Saccharosemonocarbonsäuren nach einem der Ansprüche 1 bis 4.

7. Verfahren zur Herstellung der acylierten Saccharosemonocarbonsäuren nach einem der Ansprüche 1 bis 4, **dadurch gekennzeichnet, daß** man Saccharosemonocarbonsäuren der Formel II 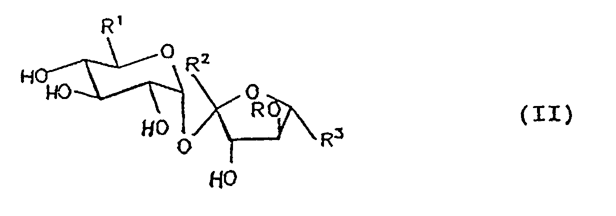 oder eines Gemisches davon in Pyridin mit Carbonsäureanhydrid oder -chlorid umsetzt, dessen Carbonsäurerest = - CO - R' ist, wobei R', R¹, R², R³ und n die in Anspruch 1 genannte Bedeutung haben.

8. Verfahren nach Anspruch 7, **dadurch gekennzeichnet, daß** der Carbonsäurerest - CO - R' ein Capronsäure-, Caprylsäure-, Caprinsäure-, Laurinsäure-, Myristinsäure-, Palmitinsäure-, oder Stearinsäurerest ist.

9. Verfahren nach Anspruch 7 oder 8, **dadurch gekennzeichnet, daß** die Umsetzung bei 0°C bis 100°C, insbesondere 20°C bis 50°C erfolgt.

10. Verfahren zur Herstellung der acylierten Saccharosemonocarbonsäuren nach einem der Ansprüche 1 bis 4, **dadurch gekennzeichnet, daß** man Saccharosemonocarbonsäuren der Formel II 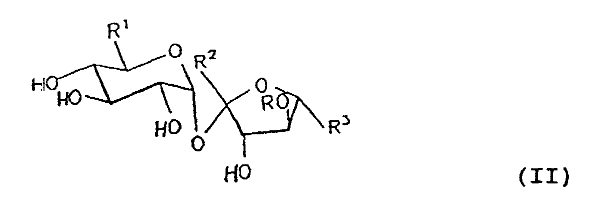 oder eines Gemisches davon lösungsmittelfrei mit Carbonsäureestern katalytisch umsetzt, deren Carbonsäurerest = - CO - R' ist, wobei R', R¹, R², R³ und n die in Anspruch 1 genannte Bedeutung haben.

11. Verfahren nach Anspruch 10, **dadurch gekennzeichnet, daß** die Carbonsäureester Methyl- oder Ethylester insbesondere der Carbonsäuren Caprinsäure, Laurinsäure, Myristinsäure, Palmitinsäure oder Stearinsäure sind.

12. Verfahren nach Anspruch 10 oder 11, **dadurch gekennzeichnet, daß** die Umsetzung bei 100°C bis 200°C, insbesondere bei 120°C bis 180°C erfolgt.

13. Verfahren nach einem der Ansprüche 7 bis 12, **dadurch gekennzeichnet, daß** ein Katalysator verwendet wird.

14. Verfahren nach einem der Ansprüche 7 bis 13, **dadurch gekennzeichnet, daß** der Katalysator 4 - (Dimethylamino)-pyridin oder Kaliumcarbonat mit Kaliumstearat ist.

15. Waschmittel, enthaltend mindestens eine der acylierten Saccharosemonocarbonsäuren. nach einem der Ansprüche 1 bis 4.

16. Verwendung der acylierten Saccharosemonocarbonsäuren nach einem der Ansprüche 1 bis 4 als grenzflächenaktive Substanz oder als Zusatz in Wasch-, Spül- und Reinigungsmitteln.

## Claims

1. Acylated sucrose monocarboxylic acids of the formula I 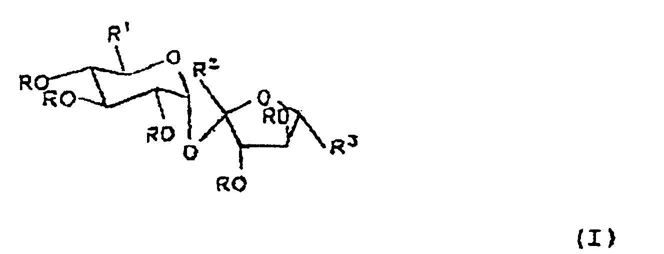 in which one of the residues R¹, R² and R³ represents COOH and the other two are CH₂OH, and R = H or CO - R', where R' = (CH₂)ₙ - CH₃ with n = 4 - 16, and at least one residue R = CO - R'.

2. Acylated sucrose monocarboxylic acids according to claim 1, **characterised in that** the acylated sucrose monocarboxylic acids have different R' residues.

3. Acylated sucrose monocarboxylic acids according to claim 1 or 2, **characterised in that** -CO - R' is a caproic acid, caprylic acid, capric acid, lauric acid, myristic acid, palmitic acid, or stearic acid residue.

4. Acylated sucrose monocarboxylic acids according to one of claims 1 to 3, **characterised in that** they have a degree of acylation of ≤ 2.

5. Aqueous solution, containing at least one of the acylated sucrose monocarboxylic acids according to one of claims 1 to 4.

6. Powder, containing at least one of the acylated sucrose monocarboxylic acids according to one of claims 1 to 4.

7. Method of producing the acylated sucrose monocarboxylic acids according to one of claims 1 to 4, **characterised in that** sucrose monocarboxylic acids of the formula II 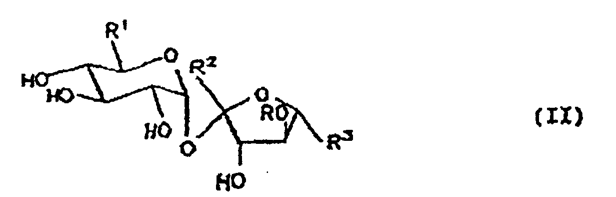 or a mixture thereof are/is reacted in pyridine with carboxylic acid anhydride or carboxylic acid chloride, the carboxylic acid residue of which = - CO - R', where R', R¹, R² and R³ and n have the meaning given in claim 1.

8. Method according to claim 7, **characterised in that** the carboxylic acid residue - CO - R' is a caproic acid, caprylic acid, capric acid, lauric acid, myristic acid, palmitic acid or stearic acid residue.

9. Method according to claim 7 or 8, **characterised in that** the reaction takes place at 0°C to 100°C, especially at 20°C to 50°C.

10. Method of producing the acylated sucrose monocarboxylic acids according to one of claims 1 to 4, **characterised in that** sucrose monocarboxylic acids of the formula II 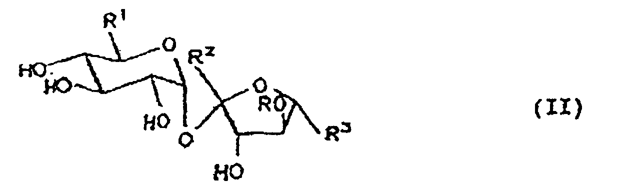 or a mixture thereof undergo/undergoes solvent-free catalytic reaction with carboxylic acid esters, of which the carboxylic acid residue = - CO - R', where R', R¹, R², R³ and n have the meaning given in claim 1.

11. Method according to claim 10, **characterised in that** the carboxylic acid esters are methyl or ethyl esters especially of the carboxylic acids capric acid, lauric acid, myristic acid, palmitic acid, or stearic acid.

12. Method according to claim 10 or 11, **characterised in that** the reaction takes place at 100°C to 200°C, especially at 120°C to 180°C.

13. Method according to one of claims 7 to 12, **characterised in that** a catalyst is used.

14. Method according to one of claims 7 to 13, **characterised in that** the catalyst is 4-(dimethylamino)pyridine or potassium carbonate with potassium stearate.

15. Detergent, containing at least one of the acylated sucrose monocarboxylic acids according to one of claims 1 to 4.

16. Use of the acylated sucrose monocarboxylic acids according to one of claims 1 to 4 as a surface-active substance or as an additive in detergents, rinsing agents or cleaning agents.

## Revendications

1. Acides monocarboxyliques de saccharose acylés de formule I : 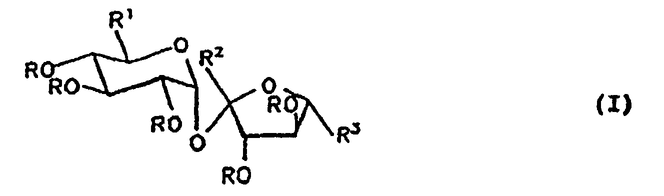 dans laquelle un des restes R¹, R² et R³ représente COOH et les deux autres restes sont CH₂OH et R signifie H ou CO - R' dans laquelle R' = (CH₂)ₙ - CH₃ avec n = 4 - 16, et dans laquelle au moins un reste R = CO - R'.

2. Acides monocarboxyliques de saccharose acylés selon la revendication 1,
**caractérisés en ce que**
les acides monocarboxyliques de saccharose acylés possèdent différents restes R'.

3. Acides monocarboxyliques de saccharose acylés selon la revendication 1 ou la revendication 2,
**caractérisés en ce que**
-CO - R' est un reste d'acide caproïque, d'acide caprylique, d'acide caprique, d'acide laurique, d'acide myristique, d'acide palmitique ou d'acide stéarique.

4. Acides monocarboxyliques de saccharose acylés selon l'une quelconque des revendications 1 à 3,
**caractérisés en ce que**
ceux-ci possèdent un degré d'acylation ≤ 2.

5. Solution aqueuse contenant au moins un des acides monocarboxyliques de saccharose acylés selon l'une quelconque des revendications 1 à 4.

6. Poudre contenant au moins un des acides monocarboxyliques de saccharose acylés selon l'une quelconque des revendications 1 à 4.

7. Procédé de préparation des acides monocarboxyliques de saccharose acylés selon l'une quelconque des revendications 1 à 4,
**caractérisé en ce qu'**
on fait réagir des acides monocarboxyliques de saccharose de formule II : 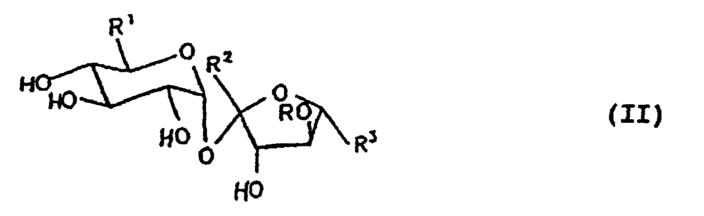 ou un mélange de ceux-ci,
dans la pyridine avec un anhydride ou un chlorure d'acide carboxylique, dont le reste d'acide carboxylique est -CO-R', avec R', R¹, R², R³ et n ayant les significations mentionnées à la revendication 1.

8. Procédé selon la revendication 7,
**caractérisé en ce que**
le reste d'acide carboxylique -CO-R' est un reste d'acide caproïque, d'acide caprylique, d'acide caprique, d'acide laurique, d'acide myristique, d'acide palmitique ou d'acide stéarique.

9. Procédé selon la revendication 7 ou 8,
**caractérisé en ce que**
la réaction s'effectue de 0 à 100°C, en particulier de 20°C à 50°C.

10. Procédé de préparation d'acides monocarboxyliques de saccharose acylés selon l'une quelconque des revendications 1 à 4,
**caractérisé en ce qu'**
on fait réagir des acides monocarboxyliques de saccharose de formule II : 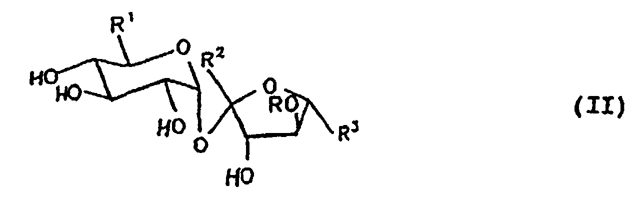 ou un mélange de ceux-ci, catalytiquement avec des esters d'acide carboxylique sans solvant, dont le reste acide carboxylique est -CO-R', avec R', R¹, R², R³ et n ayant les significations mentionnées à la revendication 1.

11. Procédé selon la revendication 10,
**caractérisé en ce que**
les esters d'acide carboxylique sont des esters méthyliques ou éthyliques, en particulier des acides carboxyliques d'acide caprique, d'acide laurique, d'acide myristique, d'acide palmitique ou d'acide stéarique.

12. Procédé selon la revendication 10 ou la revendication 11,
**caractérisé en ce que**
la réaction s'effectue de 100 à 200°C en particulier de 120°C à 180°C.

13. Procédé selon l'une quelconque des revendications 7 à 12,
**caractérisé en ce qu'**
on utilise un catalyseur.

14. Procédé selon l'une quelconque des revendications 7 à 13,
**caractérisé en ce que**
le catalyseur est la 4-(diméthylamino)-pyridine ou le carbonate de potassium avec du stéarate de potassium.

15. Produit de lavage contenant au moins un des acides monocarboxyliques de saccharose acylés selon l'une quelconque des revendications 1 à 4.

16. Utilisation des acides monocarboxyliques de saccharose acylés selon l'une quelconque des revendications 1 à 4, en tant que substance active sur la tension superficielle ou comme additif dans des produits de lavage, de rinçage et de nettoyage.
